# EUROPEAN PATENT APPLICATION

(11) **EP 2 982 404 A1**
(43) Date of publication of application: **10.02.2016**
(21) Application number: 14780019.7
(22) Date of filing: 03.03.2014
(51) Int. Cl.: A61M 25/06, A61M 5/158

(54) **STAB-PREVENTION INDWELLING NEEDLE**

(30) Priority: 03.04.2013 CN 201320165689 U
(71) Applicant: Sunwell Biotech Co., Ltd., Nantong, Jiangsu 226000 (CN)
(72) Inventor: LI, Zhiyun, Nantong Jiangsu 226000 (CN)
(74) Representative: Baldwin, Mark
(86) International application number: PCT/CN2014/072790
(87) International publication number: WO 2014/161414

(57) **Abstract**

A stab-prevention indwelling needle is disclosed. The stab-prevention indwelling needle of the present invention comprises a detained trocar, a trocar seat, a puncture needle, a protective seat, and a needle seat. The protective seat comprises a through hole, a rebound unit, and a sticking plate. When the puncture needle is withdrawn from the detained trocar and the trocar seat, the tip of the puncture needle is restricted in the protective seat by the rebound unit and the sticking plate for preventing the tip of the puncture form being exposed outside.

## Description

### [Field of the invention]

The present invention relates to a vein detained needle, particularly to a stab-prevention indwelling needle for preventing needle pricking.

### [Background of the invention]

For the prevention of pain caused by puncturing for injection several times, a vein detained needle is generally used for a patient who requires intravenous injection several times.

In general, the vein detained needle comprises a detained trocar and a puncture needle. When the vein detained needle is used clinically, the detained trocar is led by the puncture needle to enter the vein of human body. When the puncture of vein is confirmed after blood return is found, the puncture needle is withdrawn, and only the detained trocar is detained in the vein. And then, the detained trocar can be used for medicament infusion or blood extraction from the vein.

There is no protective structure for the conventional vein detained needle. The puncture needle is exposed outside after it has been withdrawn. The medical personnel may be wounded and infected by the puncture needle adhered with blood of the patient. Furthermore, the puncture needle with blood of the patient is also troublesome for medical waste disposal.

### SUMMARY OF THE PRESENT INVENTION

It is an objective of the present invention to provide a vein detained needle, more particularly a stab-prevention indwelling needle for preventing needle pricking.

It is another objective of the present invention to provide a stab-prevention indwelling needle with a protective seat for containing the tip of the withdrawn puncture needle.

The present invention provides a stab-prevention indwelling needle, comprising: a trocar seat having a front end and a back end; a detained trocar disposed at the front end of the trocar seat; a protective seat assembled at the back end of the trocar seat, wherein the protective seat comprises a through hole, a rebound unit, and a sticking plate; a needle seat having a containing space for containing the protective seat; and a puncture needle having a tip, an end portion and a holder portion disposed near the tip, wherein the end portion of the puncture needle is fixed in the needle seat, the tip of the puncture needle passes through the through hole of the protective seat, the sticking plate, the rebound unit, the trocar seat, and the detained trocar, wherein the tip of the puncture needle is restricted in the protective seat by the rebound unit, sticking plate and the holder portion after the puncture needle is withdrawn from the detained trocar and the trocar seat.

In one embodiment of the present invention, the holder portion of the puncture needle is embodied by a flat portion.

In one embodiment of the present invention, the protective seat comprises a first recess and a second recess for disposing the rebound unit and the sticking plate respectively.

In one embodiment of the present invention, the sticking plate comprises a sticking hole for sticking the holder portion of the puncture needle.

In one embodiment of the present invention, the sticking hole has a diameter slightly greater than a diameter of the puncture needle and slightly smaller than a width of the holder portion.

In one embodiment of the present invention, the rebound unit comprises a blocking plate with a blocking portion, wherein the blocking portion blocks the through hole of the protective seat when the blocking plate is free from stress.

In one embodiment of the present invention, the blocking portion of the blocking plate is away from the through hole of the protective seat when the blocking plate is bent by the puncture needle.

In one embodiment of the present invention, the rebound unit further comprises a support plate.

In one embodiment of the present invention, the trocar seat is selectively one of a straight trocar seat or a Y-shaped trocar seat.

In one embodiment of the present invention, the trocar seat is a Y-shaped trocar seat with an isolation plug, and the puncture needle comprises a blood return groove disposed between the tip and the holder portion.

### BRIEF DESCRIPTION OF THE DRAWINGS

- Fig. 1: is schematic exploded diagram of a stab-prevention indwelling needle in accordance with one embodiment of the present invention.
- Fig. 2: is another perspective partial view of the stab-prevention indwelling needle in accordance with the embodiment shown in Fig. 1.
- Fig. 3: is a schematic partial assembly diagram of the stab-prevention indwelling needle in accordance with the embodiment shown in Fig. 1.
- Fig. 4: is a schematic assembly diagram of the stab-prevention indwelling needle in accordance with the embodiment shown in Fig. 1.
- Fig. 5: is a schematic diagram of the stab-prevention indwelling needle in accordance with the embodiment shown in Fig. 1 when withdrawing the puncture needle.
- Fig. 6: is a schematic partial view and partial cross-sectional view of the stab-prevention indwelling needle in accordance with the embodiment shown in Fig. 1 when the puncture needle is withdrawn.
- Fig. 7: is a schematic diagram of the stab-prevention indwelling needle in accordance with the embodiment shown in Fig. 1 when the puncture needle is withdrawn.
- Fig. 8: is a schematic assembly diagram of a stab-prevention indwelling needle in accordance with another embodiment of the present invention.
- Fig. 9: is a partial exploded diagram of the detained needle in accordance with the embodiment shown in Fig. 8.

### DETAILED DESCRIPTION OF THE PREFERRED EMBODIMENTS

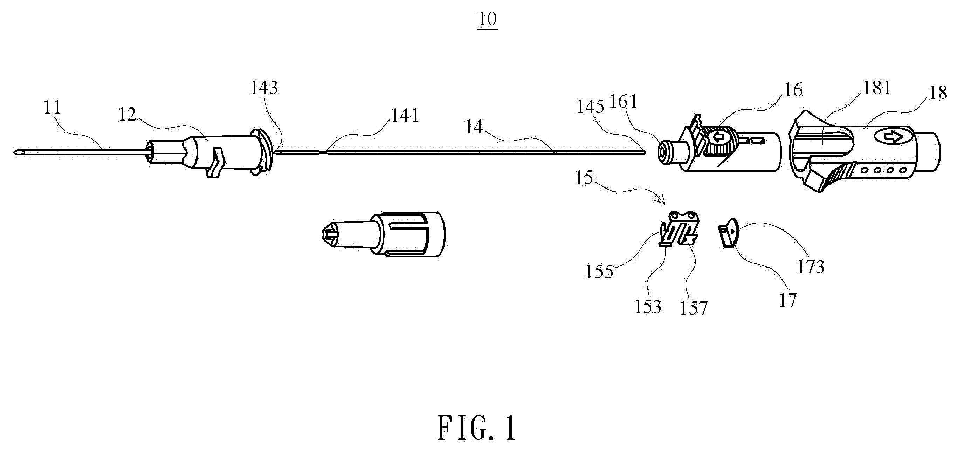

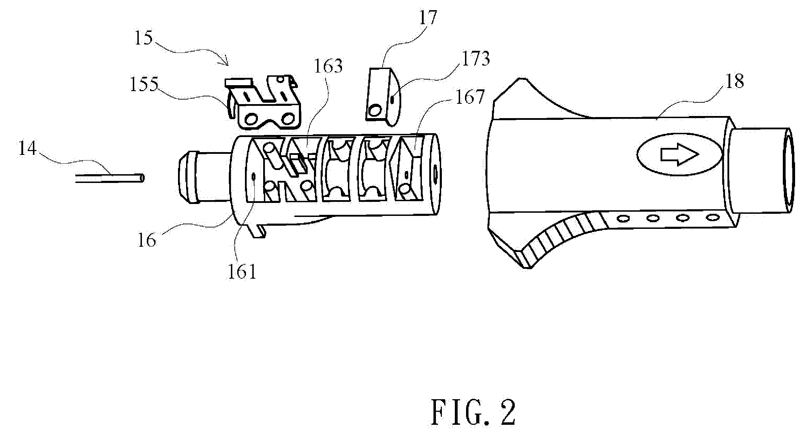

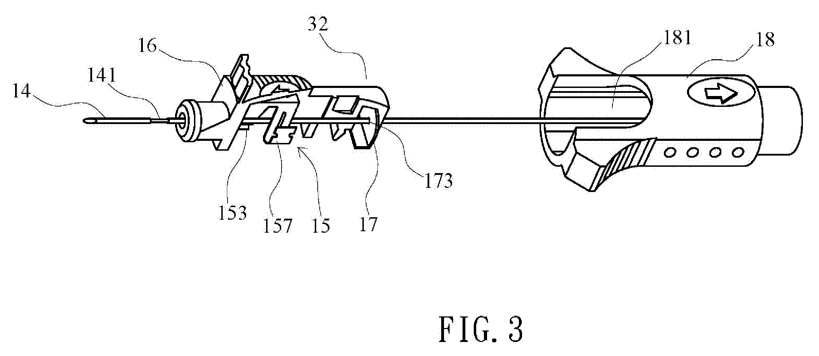

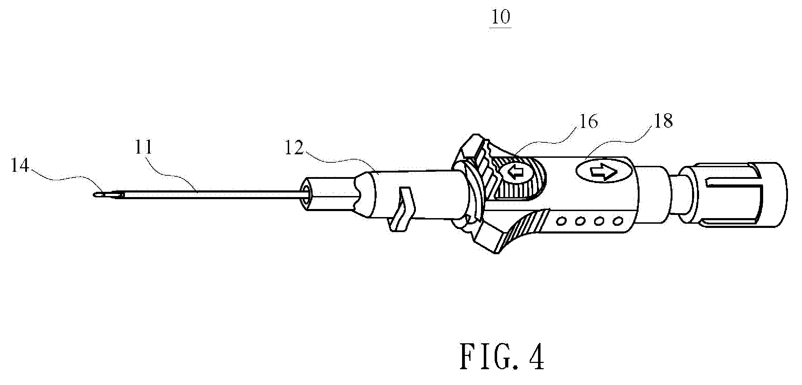

Referring to Figs. 1-4, a schematic exploded diagram, another perspective partial view, a schematic partial assembly diagram, and a schematic assembly diagram of a stab-prevention indwelling needle in accordance with one embodiment of the present invention are illustrated. In the present embodiment, the stab-prevention indwelling needle 10 comprises a detained trocar 11, a trocar seat 12, a puncture needle 14, a protective seat 16, and a needle seat 18. The detained trocar 11 is disposed at the front end of the trocar seat 12. The puncture needle 14 has a tip 143 and an end portion 145. A holder portion 141 is disposed near the tip 143 of the puncture needle 14. The protective seat 16 comprises a through hole 161, a rebound unit 15, and a sticking plate 17. The sticking plate 17 comprises a sticking hole 173. The rebound unit 15 comprises a blocking plate 153 with a blocking portion 155 disposed on the blocking plate 153. The needle seat 18 comprises a containing space 181 for containing the protective seat 16.

When assembling the stab-prevention indwelling needle 10, the end portion 145 of the puncture needle 14 is pierced through the through hole 161 of the protective seat 16 and the sticking hole 173 of the sticking plate 17 from the front end of the protective seat 16, and is fixed in the needle seat 18. Wherein the blocking plate 153 of the rebound unit 15 is bent for the blocking portion 155 to be away from the through hole 161 of the protective seat 16. The detained trocar 11 and the trocar seat 12 are slipped on the puncture needle 14 to the protective seat 16 with the tip 143 out of the detained trocar 11.

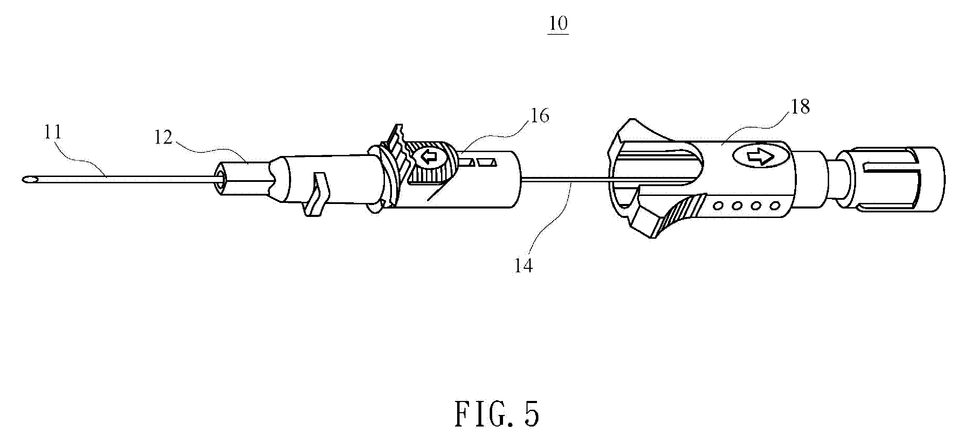

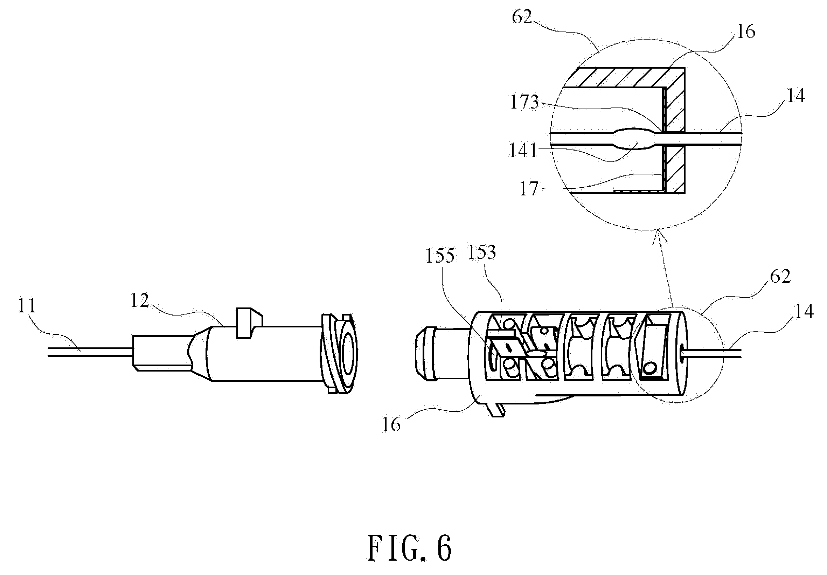

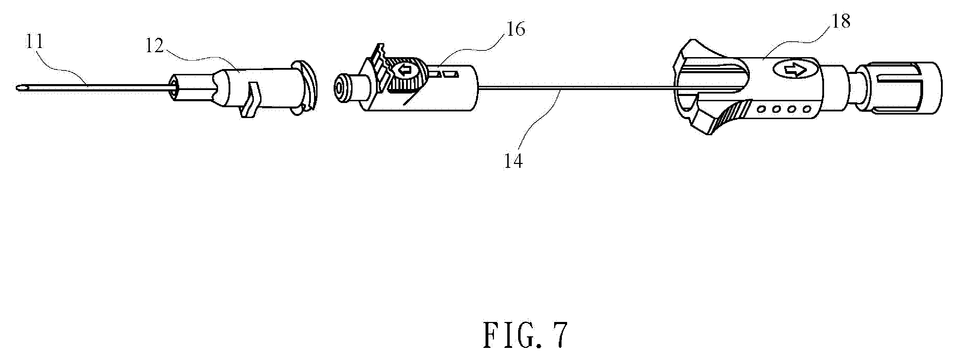

Referring to Figs. 5-7, steps for withdrawing the puncture needle of the stab-prevention indwelling needle in accordance with the embodiment shown in Fig. 1 are illustrated. When the stab-prevention indwelling needle 10 is used clinically, the detained trocar 11 is led by the puncture needle 14 to enter the vein of human body. When the puncture of vein is confirmed after blood return is found, the puncture needle 14 is withdrawn.

When withdrawing the puncture needle 14, the protective seat 16 is firstly propped to prevent the trocar seat 12 from shifting, and then the needle seat 18 is pulled backward to withdraw the puncture needle 14. When the holder portion 141 of the puncture needle 14 is pulled to the position of the sticking plate 17, the holder portion 141 will be stuck by the sticking hole 173. Besides, once the tip 143 of the puncture needle 14 is away from the blocking plate 153 of the rebound plate 15, the blocking plate 153 rebounds and the blocking portion 155 blocks the through hole 161 of the protective seat 16. This prevents the tip 143 of the puncture needle 14 from being exposed outside.

After the puncture needle 14 is withdrawn, the protective seat 16 and the trocar seat 12 are separated, only the detained trocar 11 is detained in the vein.

In one embodiment of the present invention, the diameter of the sticking hole 173 is slightly greater than the diameter of the puncture needle 14 and slightly smaller than the width of the holder portion 141.

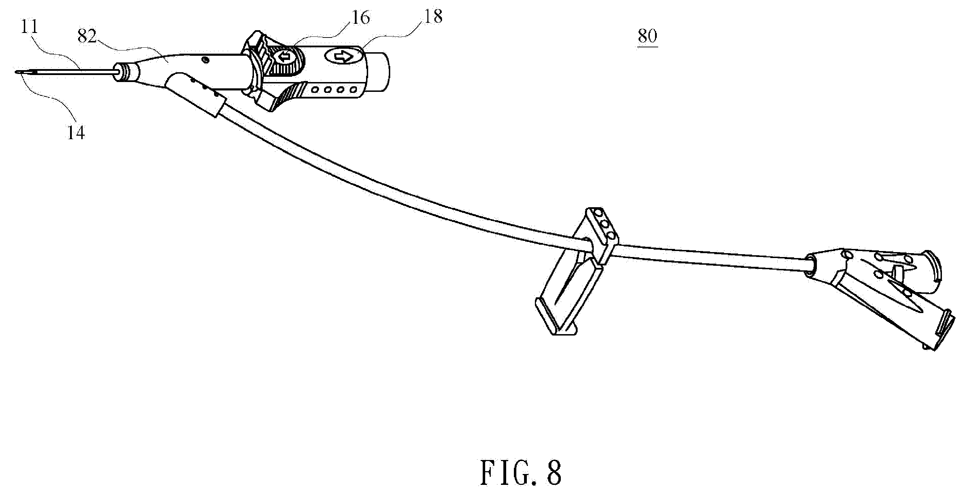

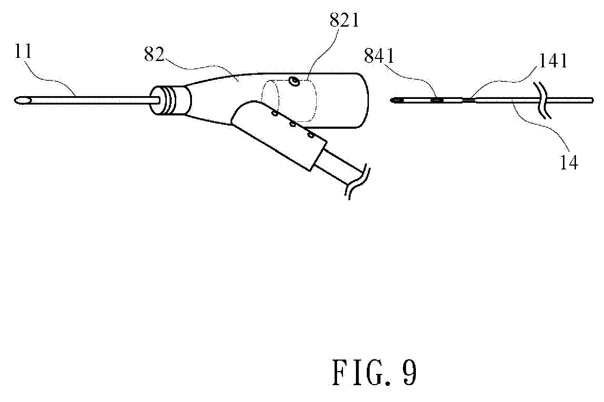

Referring to Figs. 8-9, a schematic assembly diagram and a partial exploded diagram of a stab-prevention indwelling needle in accordance with another embodiment of the present invention are illustrated. In one embodiment of the present invention, the trocar seat is selectively one of a straight trocar seat 12 (as shown in Figs. 1-7) or a Y-shaped trocar seat 82 (as shown in Figs. 8-9). In the stab-prevention indwelling needle 80 with Y-shaped trocar seat 82 of the present embodiment, the structure of the puncture needle 14, protective seat 16, and the needle seat 18 are substantially the same as the embodiment shown in Fig. 1.

The Y-shaped trocar seat 82 comprises an isolation plug 821 for preventing the blood or medicament from leaking out. The puncture needle 14 comprises a blood return groove 841 disposed between the tip 143 and the holder portion 141 for observing blood return. The end portion 145 of the puncture needle 14 is fixed and closed in the needle seat 18.

In one embodiment of the present invention, the holder portion 141 of the puncture needle 14 is embodied by a flat portion. In one embodiment of the present invention, the flat portion is formed by clamping the puncture needle 14 at a predetermined position. The flat portion with a wider width can be stuck by the sticking hole 173 of the sticking plate 17.

In one embodiment of the present invention, the protective seat 16 comprises a first recess 163 and a second recess 167 for disposing the rebound unit 15 and the sticking plate 17 respectively.

In one embodiment of the present invention, the rebound unit 15 further comprises a support plate 157 for supporting the rebound unit 15 when the blocking plate 153 is bent and preventing the rebound unit 15 from being shifted.

By using the stab-prevention indwelling needle 10 and 80, the tip 143 of the puncture needle 14 with the blood of the patient is contained in the protective seat 16. This prevents the tip 143 of the puncture needle 14 from being exposed outside. The safety of the medical personnel is ensured, and the difficulty of medical waste disposal is reduced.

Although particular embodiments of the invention have been described in detail for purposes of illustration, various modifications and enhancements may be made without departing from the spirit and scope of the invention. Accordingly, the invention is not to be limited except as by the appended claims.

## Claims

1. A stab-prevention indwelling needle, comprising:
a trocar seat having a front end and a back end;
a detained trocar disposed at the front end of the trocar seat;
a protective seat assembled at the back end of the trocar seat, wherein the protective seat comprises a through hole, a rebound unit, and a sticking plate;
a needle seat having a containing space for containing the protective seat; and
a puncture needle having a tip, an end portion and a holder portion disposed near the tip, wherein the end portion of the puncture needle is fixed in the needle seat, the tip of the puncture needle passes through the through hole of the protective seat, the sticking plate, the rebound unit, the trocar seat, and the detained trocar,
wherein the tip of the puncture needle is restricted in the protective seat by the rebound unit, sticking plate and the holder portion after the puncture needle is withdrawn from the detained trocar and the trocar seat.

2. The stab-prevention indwelling needle as claimed in claim 1, wherein the holder portion of the puncture needle is embodied by a flat portion.

3. The stab-prevention indwelling needle as claimed in claim 1, wherein the protective seat comprises a first recess and a second recess for disposing the rebound unit and the sticking plate respectively.

4. The stab-prevention indwelling needle as claimed in claim 3, wherein the sticking plate comprises a sticking hole for sticking the holder portion of the puncture needle.

5. The stab-prevention indwelling needle as claimed in claim 4, wherein the sticking hole has a diameter slightly greater than a diameter of the puncture needle and slightly smaller than a width of the holder portion.

6. The stab-prevention indwelling needle as claimed in claim 3, wherein the rebound unit comprises a blocking plate with a blocking portion, wherein the blocking portion blocks the through hole of the protective seat when the blocking plate is free from stress.

7. The stab-prevention indwelling needle as claimed in claim 6, wherein the blocking portion of the blocking plate is away from the through hole of the protective seat when the blocking plate is bent by the puncture needle.

8. The stab-prevention indwelling needle as claimed in claim 6, wherein the rebound unit further comprises a support plate.

9. The stab-prevention indwelling needle as claimed in claim 1, wherein the trocar seat is selectively one of a straight trocar seat or a Y-shaped trocar seat.

10. The stab-prevention indwelling needle as claimed in claim 9, wherein the trocar seat is a Y-shaped trocar seat with an isolation plug, and the puncture needle comprises a blood return groove disposed between the tip and the holder portion.
